# FASCICULE DE BREVET EUROPEEN

(11) **EP 2 531 531 B1**
(45) Date de publication et mention de la délivrance du brevet: **14.09.2022**
(21) Numéro de dépôt: 11707447.6
(22) Date de dépôt: 01.02.2011
(51) Int. Cl.: C08L 3/02, A61K 45/06, C12P 19/18, A61P 7/08, C08B 30/12, C08B 30/18, A61K 31/718, C12P 19/04, A61M 1/28

(54) **POLYMERES SOLUBLES DE GLUCOSE BRANCHES POUR LA DIALYSE PERITONEALE**
LÖSLICHE VERZWEIGTE GLUCOSEPOLYMERE FÜR DIE PERITONEALE DIALYSE
SOLUBLE BRANCHED GLUCOSE POLYMERS FOR PERITONEAL DIALYSIS

(30) Priorité: 02.02.2010 FR 1050736
(43) Date de publication de la demande: 12.12.2012
(73) Titulaire: Roquette Frères, 62136 Lestrem (FR)
(72) Inventeur: GUERIN-DEREMAUX, Laëtitia, F-59850 Nieppe (FR); PETITJEAN, Carole, 59160 Lomme (FR); WILS, Daniel, F-59190 Morbecque (FR)
(74) Mandataire: Plasseraud IP
(86) Numéro de dépôt international: PCT/FR2011/050201
(87) Numéro de publication internationale: WO 2011/095736

(56) Documents cités:
- EP-A1- 0 667 356
- EP-A1- 1 943 908
- EP-A2- 1 548 033
- WO-A1-03/106502
- WO-A1-2007/099212
- US-A- 3 928 135
- US-A1- 2002 065 410
- US-A1- 2005 142 167

## Description

L'invention a pour objet des polymères solubles de glucose branchés préparés à partir d'amidon présentant une teneur en amylose comprise entre 35 et 80% en poids, présentant une teneur en liaisons glucosidiques α-1, 6 comprise entre 7 et 10 %, de préférence comprise entre 8 et 9 % pour une distribution de poids moléculaire en poids (Mw) étroite, comprise entre 50.000 et 150.000 daltons.

Ces polymères solubles de glucose branchés présentent également une certaine résistance à l' α-amylase et une faible osmolalité, inférieure à 100 mOsm/kg.

Au sens de l'invention, on entend par « certaine résistance à l' α-amylase » la capacité qu'ont ces polymères solubles de glucose branchés à être peu glycémiants et à prolonger la durée de leur pouvoir osmotique dans un milieu contenant notamment de l' α-amylase (simulant l'hydrolyse de produits au sein du péritoine), autorisant ainsi leur usage notamment dans les applications en dialyse péritonéale, pour les traitements de longue durée.

Ces polymères solubles de glucose branchés présentent également un profil de distribution des longueurs de chaînes branchées tout à fait particulier.

Par « profil de distribution des longueurs de chaînes branchées » on entend, au sens de l'invention, la répartition en taille, exprimée en degré de polymérisation (ou DP), des chaînes glucosidiques linéaires α-1,4 reliées à d'autres chaînes glucosidiques linéaires α-1,4 par des points de branchement α-1,6.

L'invention vise encore des compositions comprenant de tels polymères solubles de glucose branchés utilisables dans de nombreuses applications industrielles, notamment dans les industries pharmaceutiques et plus particulièrement en dialyse péritonéale.

L'invention concerne enfin un procédé de fabrication desdits polymères solubles de glucose branchés ainsi qu'un procédé de purification desdits polymères de manière à les destiner plus particulièrement à la fabrication de solutions de dialyse péritonéale.

Les polymères de glucose classiquement fabriqués industriellement sont préparés par hydrolyse des amidons naturels ou hybrides et de leurs dérivés.

Ces hydrolysats d'amidon sont ainsi produits par hydrolyse acide ou enzymatique d'amidon de céréales ou de tubercules. Ils sont en fait un mélange de glucose et de polymères du glucose, de poids moléculaires extrêmement variés.

Ces hydrolysats d'amidon (dextrines, maltodextrines...) produits dans l'industrie (avec un certain Degré de Polymérisation ou DP moyen) comprennent une large distribution de saccharides contenant à la fois des structures linéaires (liaisons glucosidiques α-1,4) et branchées (liaisons glucosidiques **α**-1,6).

Ces hydrolysats d'amidon, et notamment les maltodextrines, sont généralement utilisés comme transporteur ou agent de charge, agent texturant, support d'atomisation, agent de remplacement de matières grasses, agent filmogène, agent de contrôle de congélation, agent anti-cristallisant, ou pour leur apport nutritionnel.

Il est connu de l'homme du métier que la composition saccharidique des maltodextrines détermine à la fois leurs propriétés physiques et biologiques.

C'est ainsi que leur hygroscopicité, leur fermentescibilité, leur viscosité, leur caractère édulcorant, leur stabilité, leur caractère gélifiant et leur osmolalité sont des critères classiquement appréciés et choisis selon leurs différents domaines d'application.

Les connaissances de base du comportement physico-chimique de ces saccharides conduisent ainsi à les intégrer par exemple dans les solutions pour dialyse péritonéale.

La dialyse péritonéale consiste à introduire une solution de dialyse dans la cavité péritonéale au moyen d'un cathéter.

Au bout d'un certain temps, un échange de solutés se produit entre le sang et le dialysat.

L'utilisation d'un agent osmotique adéquat permet le drainage de l'eau excédentaire, du sang vers le dialysat et de pallier ainsi la déficience des reins.

La méthode classique en dialyse péritonéale pour éliminer l'excès d'eau (ultrafiltration) et de solutés de l'organisme consiste à injecter dans la cavité péritonéale une solution de dialyse hypertonique par rapport au sang par addition de glucose comme agent osmotique.

Le flux à travers une membrane semi-perméable idéale est principalement déterminé par le nombre total de particules de soluté (osmolalité) présentes dans la solution, indépendamment de leur taille.

En revanche, dans le cas d'une membrane biologique telle que la membrane péritonéale, le flux dépend uniquement des solutés ne traversant pas ou peu la membrane et n'est donc pas nécessairement lié à l'osmolalité totale de la solution.

En outre, la capacité des solutés à traverser la membrane est aussi fonction de la forme des molécules, de leur charge ionique et également de leur taille.

Le choix d'un agent osmotique idéal est délicat : ce dernier doit permettre un gradient osmotique de manière à déplacer l'eau et les substances toxiques du sang vers la solution de dialyse à travers le péritoine.

Il doit également être non toxique et biologiquement inerte, tout en étant métabolisable par l'organisme, une partie de celui-ci étant assimilée dans le sang.

Il ne doit pas traverser la membrane péritonéale trop rapidement, de manière à maintenir durablement un gradient d'ultrafiltration et ne pas permettre l'accumulation de substances non dégradables, indésirables dans le sang.

Le brevet EP 207.676 enseigne ainsi que pour un usage en dialyse péritonéale continue et ambulatoire, on doit préférer des hydrolysats d'amidon formant des solutions limpides et incolores à 10 % dans l'eau, ayant un Mw de 5.000 à 100.000 daltons et un indice de polymolécularité ou Ip faible.

Cela se traduit par des compositions qui renferment majoritairement des polymères de glucose de haut poids moléculaire compris entre 5.000 et 50.000 daltons, qui ne renferment pas ou très peu de glucose ou d'oligosaccharides de DP inférieur ou égal à 3, et pas ou très peu de polymères de glucose de Mw supérieur à 100.000 daltons.

En effet, pour cette application, les monomères ou polymères de faible poids moléculaire traversant rapidement la paroi péritonéale sont sans intérêt pour la création d'un gradient de pression osmotique durable, et d'autre part, les polymères de très haut poids moléculaire, supérieur à 100.000 daltons et qui sont dénués de tout pouvoir osmotique, seraient à éviter et même à proscrire puisqu'ils risquent de rétrograder et de précipiter dans l'organisme.

Dans son brevet EP 667.356, la société Demanderesse a proposé un procédé de fabrication, à partir d'amidon waxy, d'un hydrolysat d'amidon complètement soluble dans l'eau et de faible indice de polymolécularité, inférieur à 2,8, ayant un Mw compris entre 8.000 et 22.500 daltons.

Ce procédé consiste à hydrolyser par voie acide un lait d'amidon constitué exclusivement d'amylopectine, puis à compléter cette hydrolyse acide par une hydrolyse enzymatique à l'aide d'α-amylase bactérienne, et à chromatographier cet hydrolysat sur résines cationiques fortes macroporeuses sous forme alcaline ou alcalino-terreuse.

Cet hydrolysat d'amidon particulier, encore appelé icodextrine, présente un Mw déterminé par diffusion de la lumière, comme il sera décrit ci-après, de 21.000 à 22.000 daltons.

La majorité des molécules de glucose de l'icodextrine sont des chaînes linéaires liées en **α**-1,4 (plus de 90 %) avec une petite fraction de chaînes **α**-1,4 branchées en **α**-1,6 (moins de 10 %).

L'icodextrine a permis une diminution significative de l'absorption quotidienne de glucose préalablement utilisé comme agent osmotique dans les solutions pour dialyse, amenant ainsi un avantage réel pour le traitement des patients diabétiques et/ou obèses pour lesquels la charge calorique est un facteur critique.

Les solutions de dialyse péritonéale contenant de l'icodextrine comme agent osmotique (notamment commercialisée par BAXTER HEALTHCARE Corp. sous le nom de marque EXTRANEAL^{®}) sont en général utilisées pour les longs échanges quotidiens (nocturnes en dialyse péritonéale continue ambulatoire et diurnes en dialyse péritonéale automatisée).

Cette icodextrine pourrait toutefois être encore améliorée si l'on disposait d'un agent osmotique moins glycémiant, et dont le pouvoir osmotique durerait plus longtemps, amenant un allègement significatif de la procédure du traitement de dialyse.

En effet, le rendement des dialysats étant amélioré, la fréquence de changement des poches de dialyse serait diminuée, ce qui constituerait une amélioration certaine de la qualité de vie du patient.

Ainsi, le glucide idéal en dialyse péritonéale devrait :
- être soluble dans l'eau,
- avoir une faible viscosité,
- ne pas rétrograder,
- induire une cinétique d'apparition de glucose faible dans la circulation systémique,
- être hydrolysé lentement, mais être complètement dégradé par les enzymes de l'organisme au terme de cette hydrolyse,
- exercer une pression osmotique durable.

En effet, en rapport avec ces deux derniers points, le devenir des agents osmotiques administrés en solution dans la cavité péritonéale chez des insuffisants rénaux est déterminé par leur stabilité dans le liquide péritonéal, l'importance de leur absorption dans la circulation systémique et leur vitesse d'hydrolyse par l'amylase.

Or les agents osmotiques de l'art antérieur présentent tous l'inconvénient d'être rapidement hydrolysés.

De tout ce qui précède, il résulte qu'il y a donc un besoin non satisfait de disposer de polymères de glucose présentant des propriétés remarquables, notamment en termes de stabilité, de solubilité et conférant par-là même aux produits qui les contiennent des capacités plus grandes de durée de vie, de digestibilité contrôlée, ce qui en permet tout particulièrement l'usage dans le domaine de la dialyse péritonéale.

La société Demanderesse a eu le mérite de concilier tous ces objectifs réputés jusqu'alors difficilement conciliables en imaginant et en élaborant, au prix de nombreuses recherches, de nouveaux polymères solubles de glucose branchés, préparés à partir d'amidon présentant une teneur en amylose comprise entre 35 et 80 % en poids, tels que définis dans les revendications.

Comme il sera exemplifié ci-après, la société Demanderesse a trouvé que l'obtention de polymères de glucose présentant des propriétés remarquables, notamment en termes de stabilité, de plus grande durée de vie, de digestibilité contrôlée peuvent être obtenus dès lors que l'on utilise des amidons riches en amylose comme substrat de départ.

Les polymères de glucose branchés conformes à l'invention constituent une nouvelle famille de polymères solubles de glucose, au sens où elle est bien différente de celles de l'état de la technique, y compris des autres polymères solubles de glucose hautement branchés que la société Demanderesse a déjà proposés et décrits dans ses propres demandes de brevet antérieures.

La société Demanderesse a ainsi vaincu un premier préjugé technique selon lequel, comme il est affirmé dans la demande de brevet internationale WO 2004/022602, les produits à base d'amidon qui peuvent être utilisés en dialyse péritonéale doivent présenter un degré de ramification **α**-1,6 préférentiellement compris entre 11 et 18 % et un poids moléculaire compris entre 10.000 et 200.000.

La société Demanderesse avait également considéré que seuls des polymères solubles hautement branchés (taux de branchement supérieur à 10 %) pouvaient être utilisés en dialyse péritonéale :
- dans son brevet EP 1.369.432, il s'agissait de polymères solubles de glucose hautement branchés présentant une teneur en liaisons **α**-1,6 supérieure à 10 %, de préférence comprise entre 12 et 30 % et un Mw de 30.000 à 200.000 daltons ;
- dans sa demande de brevet EP 1.548.033, il s'agissait de polymères solubles de glucose hautement branchés présentant une teneur en liaisons **α**-1,6 comprise entre 13 et 17 % et un Mw de 90.000 à 150.000 daltons ;
- dans sa demande brevet EP 1.994.061, il s'agissait de polymères solubles de glucose hautement branchés présentant une teneur en liaisons **α**-1,6 comprise entre 20 et 30 % et un Mw de 20.000 à 30.000 daltons.

Or les polymères solubles de glucose branchés conformes à l'invention préparés à partir d'amidon présentant une teneur en amylose comprise entre 35 et 80 % en poids, présentent une teneur en liaisons glucosidiques **α**-1,6 comprise entre 8 et 9 %, et un Mw compris entre 50.000 et 150.000 daltons.

Ces polymères sont caractérisés par leur remarquable résistance à l' α-amylase, que l'on détermine à l'aide d'un test qui consiste à préparer des solutions aqueuses de polymères branchés conformes à l'invention, que l'on met en contact avec une amylase d'origine pancréatique afin de simuler l'hydrolyse de produits au sein du péritoine.

La société Demanderesse a décrit par le passé, dans son brevet EP 1.177.216, des polymères solubles de glucose branchés présentant entre 2,5 et 10 % de liaisons glucosidiques **α** 1,6 et un Mw compris entre 10.000 et 10⁸ daltons.

Préparés majoritairement à partir d'amidon standard et surtout d'amidon de maïs waxy, aucun de ces composés ne présentait de si remarquables propriétés de résistance à l'**α-**amylase pancréatique, comme il sera exemplifié ci-après.

Ces polymères présentent enfin une osmolalité, déterminée selon un test « A » d'une valeur inférieure à 100 mOsm/kg.

Les polymères solubles de glucose branchés conformes à l'invention présentent une teneur de liaisons glucosidiques **α**-1,6 comprise 8 et 9 %.

Celle-ci est déterminée par analyse RMN du proton.

Le taux de branchement est alors exprimé en pourcent, correspondant à la quantité de signal du proton porté par le C 1 d'un motif anhydroglucose qui lie un autre motif anhydroglucose par une liaison **α**-1,6, lorsque l'on a donné une valeur de 100 à l'ensemble des signaux des protons glucosidiques portés par tous les C 1 des résidus glucose desdits polymères solubles de glucose.

Dans ces conditions, les polymères de glucose solubles branchés conformes à l'invention présentent une teneur en liaisons glucosidiques **α**-1,6 comprise entre 8 et 9 %.

Cette teneur en liaisons glucosidiques **α**-1,6 confère aux polymères de glucose branchés conformes à l'invention une structure particulière, en terme de degré de ramification par rapport à l'amidon dont ils sont issus.

Cette teneur en liaisons glucosidiques **α**-1,6 rend difficilement digestibles les polymères de glucose branchés selon l'invention, ce qui contribue à pouvoir les utiliser dans toutes les applications où l'on recherche une digestion ralentie.

La détermination des masses moléculaires des polymères solubles de glucose branchés conformes à l'invention est réalisée par la mesure des masses moléculaires moyennes en poids (Mw).

Cette valeur est obtenue par chromatographie d'exclusion stérique sur colonnes PSS SUPREMA 100 et PSS 5 SUPREMA 1000 montées en série et couplées à un détecteur de diffusion de la lumière.

Les polymères de glucose branchés conformes à l'invention présentent alors une valeur de Mw comprise entre 50.000 et 150.000 daltons.

Les polymères conformes à l'invention sont caractérisés par leur remarquable résistance à l'**α**-amylase, résistance déterminée selon un test développé par la société Demanderesse.

Ce test, dit test « B », permet d'évaluer la résistance des polymères à l'hydrolyse amylasique, qui est notamment un critère essentiel dans le choix d'un agent osmotique pour solution de dialyse.

Selon le test « B », les conditions opératoires pour la digestion amylasique sont les suivantes :
- Peser précisément 0,6 g de produit à tester,
- Ajouter 150 ml de tampon maléate de Na pH 7 à 0,1 mol/l,
- Agiter jusqu'à la dissolution du produit,
- Placer le flacon au bain-marie thermostaté à 37°C pendant 15 minutes, pour que la température de la solution soit de 37°C,
- Réaliser un prélèvement de 1,5 ml au temps 0 minute (prélèvement SI),
- Ajouter 0,15 g de pancréatine de porc présentant une activité équivalente à 8 USP (**α**-amylase d'origine animale),
- Incuber à 37°C au bain thermostaté sous agitation (agitateur VARIONMAG de type THP 15 au trois quarts de sa puissance d'agitation) pendant 300 minutes,
- Réaliser des prélèvements de 1,5 ml aux temps : 15, 30, 45, 60, 90, 120, 180, 240, 300 minutes,
- Arrêter la réaction enzymatique en plaçant les prélèvements dans un bain à sec à 100°C, pendant 10 minutes,
- Doser les sucres réducteurs sur les prélèvements pour étudier la vitesse d'hydrolyse,
- Déterminer les masses molaires moyennes des produits de l'hydrolyse amylasique ainsi libérés.

La méthode utilisée pour le dosage des sucres réducteurs est la méthode de SOMOGYI NELSON, qui consiste :
- dans un tube bouché, à introduire 200 microlitres d'échantillon, auquel on ajoute 200 microlitres de solution de travail (réactifs tartrate de sodium et sulfate de cuivre),
- porter à ébullition, et ajouter après refroidissement du réactif arsénomolybdique, puis de l'eau.

La solution obtenue est déposée dans une microplaque, puis on lit l'absorbance au lecteur de microplaques à une longueur d'onde de 520 nanomètres.

La valeur de référence pour la détermination des sucres réducteurs selon le test « B » est celle mesurée à 300 minutes.

Quant à la méthode utilisée pour déterminer les masses molaires moyennes des produits de l'hydrolyse amylasique ainsi libérés, il s'agit d'une méthode de chromatographie HPSEC sur colonnes Shodex OH pak - SB802 - SB803 - SB805.

Le solvant d'élution est une solution de Nitrate de sodium 0,1 M + 0,02 % azide de sodium. Le débit est de 0,5 ml/min. Les colonnes sont maintenues à une température de 30°C.

Un réfractomètre est utilisé comme module de détection, les colonnes sont étalonnées avec des pullulans de masse molaire connue.

La valeur de référence pour la détermination des masses molaires moyennes selon le test « B » est également celle effectuée à 300 minutes.

Les polymères solubles de glucose branchés de l'invention présentent alors une résistance à l'alpha amylase pancréatique exprimée selon le test « B » par :
- une teneur en sucres réducteurs, comprise entre 25 et 35 %, et
- un Mw des produits libérés compris entre 6.000 et 12.000 daltons.

Une première sous-famille de polymères solubles de glucose branchés conformes à l'invention présente une résistance à l'alpha amylase pancréatique exprimée selon le test « B » par :
- une teneur en sucres réducteurs supérieure ou égale à 25 % et inférieure à 30 %,
- un Mw des produits libérés supérieur ou égal à 9.500 et inférieur à 12.000 daltons.

Une seconde sous-famille de polymères solubles de glucose branchés conformes à l'invention présente une résistance à l'alpha amylase pancréatique exprimée selon le test « B » par :
- une teneur en sucres réducteurs supérieure ou égale à 30 % et inférieure à 35 %,
- un Mw des produits libérés supérieur ou égal à 6.000 daltons et inférieur à 9.500 daltons.

Les polymères solubles de glucose branchés conformes à l'invention présentent également des valeurs d'osmolalité particulières.

L'osmolalité d'une solution est définie comme la quantité de moles d'une substance dissoutes par kg d'eau.

La mesure de l'osmolalité des polymères solubles de glucose branchés conformes à l'invention est réalisée selon le même test « A » que celui décrit dans la demande de brevet EP 1.369.432, consistant à déterminer l'osmolalité d'une solution renfermant 100 g sec de polymères de glucose branchés conformes à l'invention placés dans 1 kg d'eau.

La mesure de l'osmolalité de cette solution est effectuée sur un osmomètre MARK 3 de FISKE TM ASSOCIATES, en suivant les spécifications du constructeur, et donne une valeur d'osmolalité inférieure à 100 mOsm/kg.

Cette valeur est en conformité avec ce que l'on se doit d'attendre d'un composé destiné à la dialyse péritonéale (des valeurs trop élevées signifient une liaison forte desdits composés avec l'eau, ce qui gène leur passage au travers de la membrane semi-perméable du péritoine).

Des valeurs d'osmolalité notablement inférieures à celles du sang (300 mOsm/kg) sont recommandées.

La première sous-famille de polymères solubles de glucose branchés conformes à l'invention telle que présentée ci-avant présente alors une osmolalité remarquablement basse, comprise entre 10 et 20 mOsm/kg.

La seconde sous-famille de polymères solubles de glucose branchés conformes à l'invention telle que présentée ci-avant présente quant à elle une osmolalité comprise entre 50 et 100 mOsm/kg.

Les polymères solubles de glucose branchés conformes à l'invention sont caractérisés enfin par leur profil de distribution des longueurs de chaînes.

La détermination de la longueur des chaînes branchées des polymères solubles de glucose branchés conformes à l'invention est réalisée en deux étapes.

Une première étape consiste à débrancher lesdits produits (hydrolyse spécifique de la liaison **α**-1,6) à l'aide d'une isoamylase bactérienne, suivie par une deuxième étape d'identification du degré de polymérisation des oligosaccharides libérés par chromatographie d'exclusion stérique (HPSEC) en comparaison avec des pullulans de taille connus.

Cette technique consiste à peser 50 mg de produit à analyser et y ajouter 3,75 ml d'eau. Après agitation de ce mélange, on ajoute 0,5 ml de DiMéthylSulfOxyde (DMSO) et on porte à ébullition sous agitation pendant 30 minutes.

On abaisse ensuite la température à 45°C et on ajoute 0,25 ml de solution tampon d'acétate de sodium 1M (préalablement amené à pH 3,5 avec de l'acide acétique).

On ajoute ensuite une isoamylase extraite de *Pseudomonas amyloderasoma* commercialisée par la société HAYASHIBARA (à raison de 59.000 U/mg) et on laisse agir à 45°C pendant 20 minutes.

On répète cet ajout 20 fois, puis on arrête la réaction par ébullition pendant 3 minutes.

La solution est ensuite déminéralisé à l'aide de résines Amberlite 200 et Amberlite IRA-67 commercialisées respectivement par les sociétés FLUKA et SIGMA.

La solution est ensuite filtrée sur filtre nylon de porosité 0,45 µm avant d'injecter sur colonne HPSEC.

La chromatographie HPSEC est paramétrée de la façon suivante (sur colonne PWXL oligo commercialisée par TSK et sur colonne SB802 + 803 + 804 + 805 commercialisées par SHODEX) :
- Volume d'injection: 200 l
- Débit: 0,5 ml/min
- Température des colonnes: 40°C
- Eluant : Nitrate de sodium 0,2 M + azide de Na 0,02 %
- Durée d'élution: 180 min

La taille des oligosaccharides libérés est déterminée par leur temps d'élution par rapport au temps d'élution de pullulans de taille connue.

Les polymères de glucose branchés conformes à l'invention présentent alors un profil de distribution des longueurs de chaînes branchées caractérisé par :
- 25 à 80 % de DP < 10,
- 2 à 10 % de DP > 30,
avec un DP moyen compris entre 4 et 12.

La première famille de polymères de glucose branchés conformes à l'invention telle que présentée ci-avant présente un profil de distribution des longueurs de chaînes branchées caractérisé par :
- 25 à 40 % de DP < 10,
- 5 à 10 % de DP > 30,
avec un DP moyen compris entre 10 et 12.

La seconde famille de polymères de glucose branchés conformes à l'invention telle que présentée ci-avant présente un profil de distribution des longueurs de chaînes branchées caractérisé par :
- 65 à 80 % de DP < 10,
- 2 à 5 % de DP > 30,
avec un DP moyen compris entre 4 et 6.

En comparaison, l'icodextrine présente un profil de distribution des longueurs de chaînes branchées caractérisé par :
- 22 % de DP < 10,
- 16 % de DP > 30
avec un DP moyen de 13.

Les polymères de glucose branchés conformes à l'invention sont ainsi caractérisés par le fait qu'ils présentent, en particulier par rapport à l'icodextrine, une répartition distincte de longueurs de chaînes (une teneur en DP < 10 significativement plus élevée pour une teneur en DP > 30 plus faible).

Cette répartition de longueurs de chaînes branchées confère ainsi aux polymères de glucose branchés conformes à l'invention une structure tout à fait particulière, faite de courtes chaînes branchées.

L'invention a également pour objet une solution pour dialyse péritonéale caractérisée en ce qu'elle comprend, en tant qu'agent osmotique, au moins un polymère soluble de glucose branché, obtenu par traitement enzymatique d' amidon présentant une teneur en amylose comprise entre 35 et 80% en poids, ayant
- une teneur en liaisons glucosidiques **α**-1,6 comprise entre 8 et 9 %,
- une masse moléculaire moyenne en poids (Mw), déterminée par diffusion de la lumière, comprise entre 50.000 et 150.000 daltons.

La solution pour dialyse péritonéale selon l'invention peut en outre comprendre des électrolytes physiologiquement acceptables, comme notamment le sodium, le potassium, le calcium, le magnésium, le chlore, de manière à éviter la perte par transfert d'électrolytes du sérum vers le péritoine.

La solution, lorsqu'elle est obtenue par dissolution dans l'eau des polymères branchés selon l'invention, doit être limpide et incolore.

Elle est exempte d'endotoxines, de peptidoglycanes et de **β-**glucanes, ainsi que de contaminants provenant de la matière première ou des préparations enzymatiques utilisées pour sa fabrication.

A cet effet, les polymères hautement branchés mis en œuvre dans ladite solution auront subi une purification de manière à ôter toute coloration ou tout contaminant indésirable tel que protéines, bactéries, toxines bactériennes, fibres, traces de métaux etc., comme il sera illustré ci-après.

La solution pour dialyse selon l'invention peut comprendre également des agents tampons (lactate, acétate, gluconate notamment) et autres additifs tels que des aminoacides, de l'insuline, des polyols tels que par exemple le sorbitol, l'érythritol, le mannitol, le maltitol, le xylitol ou les hydrolysats d'amidon hydrogénés.

L'ajout de polyols à la composition, et de préférence de polyols apyrogènes et exempts des impuretés décrites précédemment (endotoxines et autres résidus d'origine bactérienne notamment) permet d'augmenter l'osmolalité de la solution de manière plus avantageuse qu'avec le glucose ou le maltose, en raison de leur pouvoir osmotique supérieur et parce qu'ils ne sont pas réducteurs.

II est enfin également possible de compléter la solution de dialyse contenant les polymères solubles de glucose hautement branchés de l'invention avec du glucose, du maltose et/ou des polymères de glucose.

Des solutions pour dialyse péritonéale à base d'acides aminés présentent un intérêt certain dans la prévention du vieillissement accéléré du péritoine lié au glucose et à ses dérivés, même si leur coût et le risque d'un délicat contrôle de l'acidose en limite la prescription exclusive et continue.

Il est alors envisageable de composer un mélange de différentes molécules dans une même solution pour dialyse péritonéale : glucose, polymères solubles de glucose hautement branchés conformes à l'invention et acides aminés.

On préfère alors procéder à la stérilisation séparée de ces différents constituants (utilisation de poches compartimentées), afin d'éviter notamment de générer des produits de dégradation du glucose (Glucose Dégradation Products ou GDPs) ou des produits résultant de la liaison du glucose auxdits acides aminés (ou AGEs) responsables d'effets néfastes sur la stabilité de la membrane péritonéale.

La solution pour dialyse selon l'invention est par ailleurs avantageuse par rapport aux produits de l'art antérieur puisque l'agent osmotique qu'elle contient permet d'exercer une pression osmotique durable et induit une cinétique lente d'apparition de glucose, tout en étant stable à la rétrogradation, répondant ainsi aux principaux critères définis précédemment.

Pour préparer les polymères solubles de glucose branchés conformes à l'invention, un procédé comprenant la succession des étapes suivantes est mis en oeuvre :
1) éclatement et solubilisation d'un lait d'amidon présentant une teneur en amylose comprise entre 35 et 80 % en poids et une matière sèche comprise entre 5 et 25 %,
2) ajout en continu d'une enzyme de branchement du glycogène extraite de micro-organismes thermophiles, présentant une activité comprise entre 25.000 U/ml et 60.000 U/ml à une dose comprise entre 800 et 2.500 U/g sec d'amidon, à une température comprise entre 60°C et 80°C pendant une durée de 12 à 18 heures,
3) de manière facultative, traitement avec une enzyme de liquéfaction de l'amidon de type **α**-amylase ayant une activité comprise entre 100 et 150 KNU/ml à une température de 95°C, un pH compris entre 5 et 8, pendant 30 à 60 minutes, de préférence pendant 45 minutes,
4) purification des polymères solubles de glucose branchés ainsi obtenus par :
   - au moins une étape de traitement au charbon actif et/ou au noir granulaire,
   - au moins une étape de filtration stérilisante, et
   - de manière facultative, au moins une étape de traitement thermique,
5) récupération des polymères solubles de glucose branchés purifiés ainsi obtenus.

Le procédé de modification de l'amidon riche en amylose par des enzymes de branchement consiste dans un premier temps à cuire l'amidon de manière à être sous forme partiellement ou totalement gélatinisée.

Cette première étape du procédé conforme à l'invention correspond à l'éclatement et la solubilisation d'un lait d'amidon riche en amylose, cette solubilisation de l'amidon devant permettre le traitement par les enzymes de branchement.

Par « amidon riche en amylose », on entend au sens de l'invention un amidon présentant une teneur en amylose d'au moino comprise entre 35 et 80 % en poids.

Dans un mode de réalisation du procédé conforme à l'invention, il est préparé un lait d'amidon de pois d'une matière sèche comprise entre 5 et 25 %, que l'on chauffe par toute technique connue par ailleurs de l'homme du métier à une température égale ou supérieure à la température de gélatinisation de l'amidon, préférentiellement comprise entre 100 et 200°C, plus préférentiellement encore comprise entre 140 et 160°C, à une pression de 4 à 5 bars pendant 3 à 5 minutes.

Par « enzymes de branchement », on entend au sens de l'invention les enzymes de branchement choisies dans le groupe constitué par les enzymes de branchement du glycogène.

Plus particulièrement ces enzymes de branchement sont extraites d'organismes et/ou de micro-organismes thermophiles.

Après cette étape de solubilisation totale ou partielle de l'amidon, il est procédé à l'introduction en continu des enzymes de branchement dans le milieu réactionnel dans des conditions qui limitent la formation des complexes intermoléculaires.

Plus particulièrement, les conditions d'introduction des enzymes de branchement dans le milieu réactionnel sont réglées en temps et en température de manière à limiter la formation des insolubles issus de la rétrogradation de l'amidon et des associations structurées amylose-lipides.

Il est donc procédé au refroidissement de la colle d'amidon ainsi partiellement ou totalement gélatinisé, de manière à l'amener à l'optimum de température de l'enzyme de branchement choisie.

Par exemple, si l'on choisit l'enzyme de branchement du glycogène thermostable extraite de microorganismes thermophiles du genre *Bacillus* (*B. stearothermophilus, B. megaterium*...), il faut amener la colle d'amidon à la température de l'optimum de fonctionnement de l'enzyme, i.e. comprise entre 60 et 80°C.

Il sera avantageusement choisi de refroidir rapidement la colle d'amidon partiellement ou totalement gélatinisé, de sa température initiale comprise entre 140 et 160°C jusqu'à une température de 60 à 80°C, pendant une durée inférieure à 15 minutes, permettant un ajout immédiat de l'enzyme de branchement pour éviter la rétrogradation de l'amidon ou la formation des associations structurées amylose-lipides.

Il est ensuite procédé à l'ajustement du pH de la solution, de manière à l'amener à une valeur conforme au mode de fonctionnement de ladite enzyme.

Comme il sera exemplifié ci-après, après l'étape d'abaissement rapide de la température du milieu réactionnel à la température de l'optimum de fonctionnement de l'enzyme de branchement, il est choisi d'utiliser ladite enzyme de branchement à une activité comprise entre 25.000 U/ml et 60.000 U/ml à une dose comprise entre 800 et 2.500 U/g sec d'amidon de pois, à une température comprise entre 60°C et 80°C pendant une durée de 12 à 18 heures.

A la fin de la réaction, il peut enfin être procédé à la désactivation thermique de l'enzyme, à une température de 90°C à 95°C.

Les polymères solubles de glucose branchés sont ainsi obtenus en solution aqueuse.

La troisième étape facultative du procédé conforme à l'invention consiste à faire agir une enzyme de liquéfaction de l'amidon telle une **α**-amylase.

Les conditions réactionnelles (température et pH) sont les suivantes : de 0,10 à 0,30 ml d' **α**-amylase, par exemple de type TERMAMYL^{®} 120L de NOVOZYMES ayant une activité comprise entre 100 et 150 KNU/ml à une température de 95°C, un pH compris entre 5 et 8, pendant 30 à 60 minutes, de préférence pendant 45 minutes.

L' α-amylase est ensuite désactivée par abaissement du pH à 3,5, par exemple à l'aide d'HCl à 10 %.

Au terme de ce traitement, les polymères solubles de glucose branchés conformes à l'invention sont obtenus en solution aqueuse, en mélange avec leurs produits de dégradation enzymatiques.

Contrairement aux enseignements des demandes de brevet précitées, notamment l'EP 1.369.432 et l'EP 1.548.033, il n'est pas obligatoirement effectué ici de fractionnement à l'aide d'une technique choisie par exemple dans le groupe des séparations sur membrane et des chromatographies, de manière à récupérer les fractions de haut poids moléculaire et à éliminer les fractions de bas poids moléculaire.

C'est ainsi que le produit direct du traitement à l'aide de l'enzyme de branchement seule, ou de l'enzyme de branchement puis de l' **α**-amylase, constituent les deux familles de polymères solubles de glucose branchés précédemment décrites :
- la première famille correspondant aux polymères de glucose conformes à l'invention obtenus par le seul traitement à l'enzyme de branchement ;
- la seconde famille correspondant aux polymères de glucose conformes à l'invention obtenus par le traitement conjoint à l'enzyme de branchement et à l'**α** amylase.

Le fractionnement des produits de la seconde famille telle que décrite ci-avant, à l'aide d'une technique choisie par exemple dans le groupe des séparations sur membrane et des chromatographies, conduirait alors à une troisième famille de polymères solubles de glucose branchés conformes à l'invention.

La quatrième étape du procédé conforme à l'invention consiste à purifier les polymères solubles de glucose branchés ainsi obtenus par :
- au moins une étape de traitement au charbon actif et/ou au noir granulaire,
- au moins une étape de filtration stérilisante, et
- de manière facultative, au moins une étape de traitement thermique.

La société Demanderesse, en combinant ces étapes de traitement au charbon actif et/ou noir granulaire, de filtration, voire de traitement thermique, dans un agencement particulier, réussit ainsi de façon remarquable à assurer une « quasi absence » de contamination, notamment d'endotoxines, de **β-**glucanes et de peptidoglycanes.

Le caractère sécurisé d'un tel procédé permet ainsi de limiter les contrôles bactériens, au terme dudit procédé, au seul test de détection des peptidoglycanes (par exemple le test de haute sensibilité mis au point et validé par la société Demanderesse - qui sera décrit ci-après), et au test de détection classique des endotoxines (test LAL = test de détection des endotoxines bactériennes, composants majoritaires des bactéries Gram négatives).

Par « quasi-absence », on entend au sens de l'invention une quantification à des seuils bien inférieurs à ce qui est décrit dans les tests de Pharmacopées, i.e. :
- pour les endotoxines (et **β** glucanes) via le test LAL (méthode gel clot en point final) utilisant des réactifs fabriqués par CHARLES RIVER-ENDOSAFE (Lysat LAL de sensibilité 0,015 E.U/ml réf. OR15015 et endotoxines CSE 500 ng ou 10 ng par flacon réf. E110 ou E120) : • 0.6 EU/g
- pour les peptidoglycanes (et **β**-glucanes) via un test de haute sensibilité mis au point par la société Demanderesse : < 8 ng/g de polymères de glucose (ainsi bien inférieur au seuil de référence décrit dans le brevet EP 1.720.999 pour les peptidoglycanes).

Par « test de haute sensibilité mis au point et validé par la société Demanderesse », on entend un test développé et validé par la société Demanderesse, en adaptant le kit SLP-HS single set réf. 293-58301 fabriqué et commercialisé par la société WAKO Pure Chemical Industries Ltd.

Ce test consiste à additionner le réactif dit « SLP-HS » (Silkworm Larvea Plasma-High sensitivity) réactif préparé à partir du plasma de ver à soie, capable de :
- réagir avec les peptidoglycanes et **β**-glucanes contenus dans une solution de polymère de glucose préparée à 5 % dans de l'eau (eau spéciale pour test LAL par exemple),
- induire une réaction en cascade de sérine-protéase et
- de détecter et/ou de quantifier lesdits peptidoglycanes et **β**-glucanes au moyen d'un lecteur de tubes TOXINOMETER fabriqué et commercialisé par la société WAKO Pure Chemical Industries Ltd à des seuils très faibles, i.e. :
   - une limite de détection (LD) à un seuil d'environ 0,05 ng/ml (soit 1 ng/g de polymère de glucose) et
   - une limite de quantification (LQ) à un seuil d'environ 0,15 ng/ml (3 ng/g de polymère de glucose).
(LD et LQ déterminées dans le produit polymère de glucose testé)

De manière plus précise, le test SLP-HP consiste à :
- préparer le polymère de glucose à tester en solution à 5 % dans de l'eau de qualité adéquate (eau spéciale pour test LAL par exemple),
- réaliser une gamme étalon de peptidoglycanes dans l'eau sur le domaine d'application de 0,04 à 2,5 ng/ml (valeurs cibles) avec le standard de peptidoglycanes (extrait de *Staphylococcus Aureus)* du kit SLP-HS single set pour l'établissement d'une droite d'étalonnage (régression linéaire en échelle logarithmique Ta = f(teneur en PG)),
- introduire 100 µl de la solution préparée à tester dans le tube HS-SLP après reconstitution par ajout de 100 µl du diluant (fourni dans le kit précédemment cité),
- introduire le tube SLP-HS dans le puits d'incubation du lecteur de tube TOXINOMETER (WAKO Pure Chemical Ltd) thermostaté à 30°C et paramétré selon les conditions prescrites par le fabricant.

La teneur en PG de la solution à tester est calculée au moyen de la droite d'étalonnage établie.

Le résultat est exprimé en ng/ml de solution à 5 % testée puis en ng/g de polymère de glucose.

Il est remarquable que les quantités de ces peptidoglycanes/ **β** glucanes dans le polymère de glucose au final obtenu grâce au procédé conforme à l'invention sont garanties bien inférieures à 8 ng/g de polymère de glucose.

Dans le procédé de purification des polymères solubles de glucose branchés destinés à la dialyse péritonéale, comme il a été décrit plus haut, la première étape de cette purification consiste à utiliser du charbon actif et/ou du noir granulaire dans une configuration particulière.

La société Demanderesse recommande de mettre en œuvre ce moyen dans au moins une des trois variantes suivantes :
- en première variante du procédé conforme à l'invention : dans le cas de l'utilisation du noir granulaire, cette configuration repose sur un fonctionnement à contre-courant.

Le temps de séjour dans la colonne est d'environ trois heures. La percolation se fait à une vitesse de l'ordre de 2 m/h à une température de l'ordre de 80°C pour éviter la contamination bactérienne.

Le contact entre les polymères de glucose conformes à l'invention à purifier avec le noir granulaire se fait à contre-courant au sens où la solution de polymères de glucose entre tout d'abord en contact avec le noir granulaire saturé en bas de colonne.

Les polymères de glucose purifiés sont donc récupérés au sommet de la colonne de noir granulaire, là où le noir granulaire purifié est ajouté à contre-courant.

De cette manière, la dernière couche de noir granulaire en haut de colonne sert de « barrière de sécurité ».

On peut contrôler cet arrangement en mettant en œuvre des opérations de « chasse » de noir granulaire. La colonne est stoppée, on soutire par le bas le noir granulaire saturé, que l'on remplace par le haut avec du noir granulaire régénéré.

Le noir granulaire saturé est désucré avant d'être régénéré par traitement thermique en four à sole.

Au démarrage, et par sécurité, les premiers m³ de polymères solubles de glucose branchés de basses matières sèches sont déclassés.

Le suivi de l'abaissement du taux de contaminants (endotoxines, peptidoglycanes et **β**-glucanes) peut être analysé en faisant un certain nombre de prélèvements (cinq par exemple) du bas de la colonne vers le haut.
- en deuxième variante du procédé conforme à l'invention : dans le cas de l'utilisation du charbon actif, cette configuration repose sur un traitement au charbon actif « en double ».

Les polymères solubles de glucose branchés entrant sont mélangés avec du charbon actif (entre 0,5 % et 5%, de préférence entre 0,5 et 1,5 % sur la matière sèche à traiter) à une température comprise entre 70 et 75°C pendant une heure.

Les polymères de glucose sont ensuite filtrés et analysés.

Les polymères de glucose subissent alors un traitement de même nature. Ce deuxième traitement est le traitement dit « de sécurité ».

La société Demanderesse recommande d'utiliser dans ces deux étages du charbon actif de porosité différente, de manière à prendre en compte la variabilité de la taille des contaminants.
- en troisième variante du procédé conforme à l'invention, il est choisi de combiner un étage de noir granulaire et un étage de charbon actif.

La société Demanderesse recommande alors de placer la colonne de noir granulaire en tête de cette combinaison.

Les conditions de mise en œuvre de ces deux étages sont conformes à ce qui est décrit ci-avant.

Le deuxième des trois moyens mis en œuvre pour purifier les polymères de glucose destinés à la fabrication de solutions de dialyse péritonéale conforme à l'invention consiste à utiliser une filtration stérilisante.

Cette étape de filtration stérilisante consiste principalement en une filtration membranaire de diamètre de pore de 0,1 µm, précédé le cas échéant d'une pré-filtration membranaire d'un diamètre de pore de 0,22 µm, elle-même précédée d'une filtration membranaire d'un diamètre de pore de 0,45 µm.

Cette étape permet de retenir toute contamination par des microorganismes, et notamment les bactéries acidothermophiles de type *Alicyclobacillus acidocaldarius,* leur taille étant supérieure aux diamètres des pores de filtration.

La filtration est réalisée par plusieurs filtres à cartouches insérées dans un carter vertical vers lequel le sirop est dirigé.

Ces filtres à cartouches sont fournis par les sociétés PALL ou MILLIPORE par exemple. La taille des cartouches peut être de 10, 20 ou 30 pouces, et le nombre de cartouches installées permet d'obtenir une surface de filtration suffisante afin de passer un débit de produit entre 1 et 20 l/minutes/m².

Ces filtres à cartouches ont des capacités de résistance pour un travail en continu à haute température, de l'ordre de 75°C et pour passer le débit précédemment cité durant une période supérieure de 700 h.

Le fait de travailler à une température supérieure à 75°C permet de limiter tout développement microbiologique, notamment des flores thermophiles.

Leur résistance à la température permet également de réaliser une stérilisation avant leur mise en service. Cette stérilisation consiste à faire passer de la vapeur 2 bars à travers le carter pendant une période de 20 minutes. Cette stérilisation est suivie par un rinçage à l'eau purifiée (au sens de la Pharmacopée) pendant une période de 5 minutes.

Ces filtres possèdent également des capacités de résistance à certains produits chimiques utilisés pour les opérations de nettoyage des équipements, et notamment l'acide peracétique à une concentration de 5 ‰.

Un test d'intégrité est réalisable sur ces cartouches à l'aide d'un intégritest de la société MILLIPORE par exemple. Ce test d'intégrité est réalisé à la mise en place des cartouches afin d'en vérifier le montage. Ce test est ensuite réalisé avant chaque nettoyage des équipements et enfin avant le démontage afin de valider leur bon fonctionnement durant la phase de production.

Le delta de pression (ΔP) de travail de ces filtres ne doit pas dépasser les 2 bars afin de garantir leur intégrité. Si tel est le cas, ces filtres doivent être remplacés par de nouveaux.

Un troisième et dernier moyen mis en œuvre pour purifier les polymères solubles de glucose branchés destinés à la fabrication de solutions de dialyse péritonéale conforme à l'invention peut consister à utiliser un traitement thermique particulier, qui sera avantageusement mis en œuvre au terme de ces étapes de purification.

La société Demanderesse a constaté que ce traitement n'est pas indispensable pour garantir le niveau de purification élevé des polymères de glucose tel qu'assuré par le procédé selon à l'invention, mais peut-être mis en œuvre par sécurité.

Plus particulièrement, ce traitement thermique consiste à régler le couple temps / température de manière à éliminer la biocharge résiduelle en microorganismes thermophiles susceptibles de contaminer encore lesdits polymères de glucose.

Cette étape de traitement thermique consiste alors à chauffer à une température comprise entre 100 et 130°C, de préférence à une température de 120°C, pendant 1 à 5 minutes, de préférence 2 minutes.

Ce traitement thermique est réalisé à l'aide d'un échangeur tubulaire dans lequel la solution de polymères de glucose circule, échangeur tubulaire entouré d'une calandre alimentée par de la vapeur 2 bars afin d'y réguler une température de l'ordre de 120°C.

Cet échangeur tubulaire est par exemple fabriqué par la société ACTINI, constitué de plusieurs parties :
- une section de récupération d'énergie produit/produit entre l'entrée et la sortie de la zone,
- une section de chauffage à l'aide de vapeur 2 bars,
- une section de chambrage intégrée et modulable en fonction du temps de séjour souhaitée.

La longueur de cet échangeur est calculée afin de garantir le temps de séjour souhaité suivant le débit d'alimentation. Par exemple le débit d'alimentation peut être compris entre 3000 et 4000 litres/heure pour une section de chambrage de l'ordre de 100 à 130 litres.

Pour illustrer ce traitement de purification des polymères de glucose conformes à l'invention, un des procédés préférés consiste, après l'étape d'inactivation de l'enzyme de branchement telle que précisée au terme de l'étape 2), ou après l'étape d'inhibition de l'**α**-amylase telle que préconisée au terme de l'étape facultative 3), de réaliser la succession des étapes suivantes :
i) amener le pH de la solution aqueuse contenant les polymères solubles de glucose branchés à une valeur comprise entre 4 et 5, de préférence à une valeur de 4,5,
ii) réaliser sur ladite solution ainsi obtenue deux étapes successives de traitement au charbon actif et/ou au noir granulaire,
iii) filtrer sur filtre de diamètre de pore de 10 µm, puis sur filtre de diamètre de pore de 1 µm,
iv) évaporer, concentrer et déminéraliser la solution de polymères solubles de glucose branchés,
v) réaliser une troisième étape de traitement au charbon actif et/ou au noir granulaire,
vi) filtrer sur filtre de diamètre de pore de 10 µm, puis sur filtre de diamètre de pore de 1,2 µm,
vii) mettre en œuvre une filtration stérilisante consistant en deux filtrations membranaires de diamètre de pore de 0,45 µm puis 0,22 µm.

La société Demanderesse recommande, au niveau de l'étape 2), entre les deux successions de traitement au charbon actif et/ou au noir granulaire, de débarrasser des particules et impuretés la solution issue du premier traitement (par exemple particules de charbon ou noir granulaire contaminant), ceci afin d'éviter le colmatage des colonnes de charbon actif et/ou de noir granulaire utilisées ensuite.

Cette élimination de cette charge minérale, voire organique peut être réalisée en conditions stériles par tout moyen connu par ailleurs de l'homme du métier.

Par exemple, par l'utilisation d'une séparatrice centrifuge à assiettes stérile, comme celle commercialisée par la société alpha LAVAL (type BTUX), par l'utilisation d'un module de microfiltration comme celui commercialisé par la société PALL et muni de membranes céramiques MEMBRALOX ou par l'utilisation d'un filtre poche type EATON de diamètre de pores successifs de 50, 25, 10µm, comme il sera exemplifié ci-après.

Cette succession d'étapes permet ainsi de sécuriser, pas à pas, l'obtention des polymères de glucose destinés à la préparation de solution pour dialyse péritonéale.

De façon tout à fait avantageuse, ces polymères solubles de glucose branchés, même en présence de leurs co-produits, sont tout à fait adaptés à l'application en dialyse péritonéale à laquelle ils sont destinés.

D'autres caractéristiques et avantages de l'invention apparaîtront à la lecture des exemples non limitatifs décrits ci-dessous.

### Exemple 1. Préparation des polymères solubles de glucose branchés de l'invention par la seule enzyme de branchement (première famille de polymères conforme à l'invention).

On prépare une solution d'amidon à 11 % de matière sèche à partir d'un amidon de pois présentant une richesse en amidon supérieure à 95 % et une teneur en amylose de 36,7 %.

Pour cela, on remet en suspension 110 g sec d'amidon de pois dans 890 ml d'eau à température ambiante et sous agitation.

On ajuste le pH à 7,0 avec de la soude à 4 %.

La solubilisation totale de l'amidon est réalisée dans un cuiseur de type JET COOKER à 150°C pendant 4 minutes sous une pression de 4 - 5 bars, puis refroidissement en flash atmosphérique à 70°C par recirculation dans un échangeur.

On ajoute en continu l'enzyme de branchement du glycogène purifiée de *Bacillus stearothermophilus* à raison de 3 ml de solution d'enzyme à 45.000 U/ml pour 100 g sec de substrat.

La réaction enzymatique est menée pendant 15 heures à 70°C et à pH 6,8 puis stoppée par chauffage à 90°C pendant 1 h.

Le milieu réactionnel est ensuite ramené à une température de 70°C et à un pH de 4.5.

Il est ensuite traité sur noir de type Norit SX+ 1% s/s pendant 1 heure et filtré sur des filtres poches type EATON de diamètre de pores successifs de 50, 25, 10 µm.

La solution ainsi obtenue subie de nouveau un traitement noir de type Norit SX+ 1 % en poids sec/sec (s/s) pendant 1 heure à 70°C puis filtrée sur filtre Cricket de porosité 11 µm puis sur filtre EATON 1 µm.

On concentre à une matière sèche de l'ordre de 30 % sur un évaporateur à flot tombant de type NIRO.

On déminéralise par passage sur lit mixte à une température inférieure à 40°C et à un débit de 3 V/V/h.

La solution déminéralisée est chauffée à 70 °C et subit un troisième traitement noir pendant 1 heure avec du Norit SX+ à 1% s/s, puis est filtrée sur filtre Cricket de diamètre de pores de 11 µm puis sur cartouches Millipore de diamètre de pore de 1.2 µm suivie d'une filtration stérilisante sur membrane de diamètre de pores 0.45 µm puis 0.22 µm.

La solution est ensuite atomisée.

Le polymère de glucose ainsi purifié possède une teneur en endotoxines < 0.3 EU/g et une teneur en peptidoglycanes < 20 ng/g ainsi qu'une absence par g de germes aérobies levures moisissures et bactérie acidophile thermophile).

Le tableau I suivant présente les résultats des caractéristiques physico-chimiques du polymère soluble de glucose branché conforme à l'invention ainsi obtenu, en comparaison avec l'icodextrine.

**Tableau I**

| | Polymères conformes à l'invention | | Icodextrine |
|---|---|---|---|
| Taux de liaisons α-1,6 | 8, 7 | 8, 4 | 7 - 7,5 |
| Mw (daltons) par diffusion de la lumière (Da) | 127.000 | 144.000 | 22.000 - 21.500 |
| Teneur en sucres réducteurs selon le test « B » (%) | 30 | 27 | 34 - 38 |
| Mw des produits libérés par GPC Nitrate(Da) | 10.375 | 9.935 | 2.700 - 3.435 |
| Osmolalité (mOSm/kg) | 13 | 13 | 10 |
| Répartition des longueurs de chaînes | | | |
| DP < 10 | 30,1 | 36,0 | 21,9 |
| DP > 30 | 7,7 | 6,1 | 16,2 |
| DP moyen | 11,4 | 10, 7 | 12,7 |

Les polymères de glucose selon l'invention présentent des pouvoirs glycémiant et osmotique remarquables en regard de ceux développés par l'Icodextrine.

Pour une osmolalité équivalente, les polymères selon l'invention expriment en effet une résistance amylasique supérieure à celle de l'icodextrine.

Il est à noter que les polymères de glucose branchés de l'invention diffèrent également de ceux préparés selon l'enseignement du brevet EP 1.177.216 dont la société Demanderesse est la titulaire.

Ce brevet EP 1.177.216 enseignait le traitement préférentiel par l'enzyme de branchement d'un amidon ou un dérivé d'amidon waxy (riche en amylopectine) en batch, et à 37°C.

La société Demanderesse a ainsi trouvé que les polymères de glucose obtenus selon ce brevet EP 1.177.216 présentent un comportement moins avantageux quant au test de résistance amylasique, ce qui se traduit notamment par des produits libérés présentent un Mw < 6.000 daltons, de préférence compris entre 5.000 et 6.000 daltons, soit de taille deux fois inférieure aux Mw des produits libérés par les produits fabriqués selon cet exemple 1.

Ces polymères solubles de glucose branchés constituent donc le meilleur compromis en termes de poids moléculaire, structure branchée et comportement quant à leur résistance amylasique, ce qui les destine plus particulièrement à leur utilisation en dialyse péritonéale.

### Exemple 2. Préparation des polymères solubles de glucose branchés de l'invention par l'enzyme de branchement couplée à une α-amylase (seconde famille de polymères conformes à l'invention).

On prépare une solution d'amidon à 11 % de matière sèche à partir d'un amidon de pois présentant une richesse en amidon supérieure à 95 % et une teneur en amylose de 36,7 %.

Pour cela, on remet en suspension 110 g sec d'amidon de pois dans 890 ml d'eau à température ambiante et sous agitation.

On ajuste le pH à 7,0 avec de la soude à 4 %.

La solubilisation totale de l'amidon est réalisée dans un cuiseur de type JET COOKER à 150°C pendant 4 minutes sous une pression de 4 - 5 bars, puis refroidissement en flash atmosphérique à 70°C par recirculation dans un échangeur.

On ajoute en continu l'enzyme de branchement du glycogène purifiée de *Bacillus stearothermophilus* à raison de 2 ml de solution d'enzyme à 45.000 U/ml pour 100 g sec de substrat.

La réaction enzymatique est menée pendant 15 heures à 70°C et à pH 6,8.

Un traitement complémentaire est réalisé avec l' **α**-amylase dans les conditions suivantes :
On ajoute 70 ppm sec/sec d'ions calcium sous forme CaCl₂.
On chauffe à 95°C.

On ajoute 0,15 % V / sec de TERMAMYL^{®} 120L à la concentration de 120 KNU/g.

L'incubation est réalisée pendant 45 minutes, et la réaction est stoppée par abaissement du pH à 3,5 avec de l'HCl à 10 % puis la solution est maintenue à pH 3,25 et à une température de 95°C pendant 15 minutes.

Le milieu réactionnel est ensuite ramené à une température de 70°C et à un pH de 4.5 puis traité sur noir de type Norit SX+ 1% s/s pendant 1 heure puis filtré sur des filtres poches type EATON de diamètre de pores successifs de 50, 25, 10 µm. La solution ainsi obtenue subit de nouveau un traitement noir de type Norit SX+ 1 % s/s pendant 1 heure à 70°C puis est filtrée sur filtre Cricket de porosité 11 µm, puis sur filtre EATON 1 µm.

On concentre à une matière sèche de l'ordre de 30 % sur un évaporateur à flot tombant de type NIRO.

On déminéralise par passage sur lit mixte à une température inférieure à 40°C et à un débit de 3 V/V/h.

La solution déminéralisée est chauffée à 70 °C et subit un troisième traitement au noir pendant 1 heure avec du Norit SX+ à 1% s/s, puis est filtrée sur filtre Cricket de diamètre de pores de 11 µm puis sur cartouches Millipore de diamètre de pore de 1.2 µm, suivie d'une filtration stérilisante sur membrane de diamètre de pores 0.45 µm puis 0.22 µm.

La solution est ensuite atomisée.

Le polymère de glucose ainsi purifié possède une teneur en endotoxines < 0.3 EU/g et une teneur en peptidoglycanes < 20 ng/g, ainsi qu'une absence par g de germes aérobies levures moisissures et bactérie acidophile thermophile.

Le tableau II suivant présente les résultats des caractéristiques physico-chimiques du polymère soluble de glucose branché conforme à l'invention ainsi obtenu, en comparaison avec l'icodextrine.

**Tableau II**

| | Polymère conforme à l'invention | | Icodextrine |
|---|---|---|---|
| Taux de liaisons α-1,6 | 8, 7 | 8,8 | 7 - 7,5 |
| Mw (daltons) par diffusion de la lumière (Da) | 58.400 | 55.000 | 22.000 - 21.500 |
| Teneur en sucres réducteurs selon le test « B » (%) | 33 | 32 | 34 - 38 |
| Mw des produits libérés par GPC Nitrate (Da) | 6.660 | 6.970 | 2.700 - 3.435 |
| Osmolalité (mOSm/kg) | 65 | 87 | 10 |
| Répartition des longueurs de chaînes | | | |
| DP < 10 | 72,2 | 75,6 | 21,9 |
| DP > 30 | 2,8 | 2,5 | 16,2 |
| DP moyen | 5,1 | 4,7 | 12,7 |

Les polymères de glucose obtenus par cette variante du procédé selon l'invention présentent eux-aussi un comportement tout à fait remarquable en regard du test de résistance à l'**α**-amylase et notamment quant au Mw des produits libérés.

## Revendications

1. Procédé de préparation des polymères solubles de glucose branchés, **caractérisé par le fait qu'**il comprend la succession d'étapes suivantes :
1) éclatement et solubilisation d'un lait d'amidon présentant une teneur en amylose comprise entre 35 et 80 % en poids, et une matière sèche comprise entre 5 et 25 %,
2) ajout en continu d'une enzyme de branchement du glycogène extraite de micro-organismes thermophiles, présentant une activité comprise entre 25.000 U/ml et 60.000 U/ml, à une dose comprise entre 800 et 2.500 U/g sec d'amidon, à une température comprise entre 60°C et 80°C, pendant une durée de 12 à 18 heures,
3) de manière facultative, traitement avec une enzyme de liquéfaction de l'amidon de type α-amylase ayant une activité comprise entre 100 et 150 KNU/ml à une température de 95°C, un pH compris entre 5 et 8, pendant 30 à 60 minutes, de préférence pendant 45 minutes,
4) purification des polymères solubles de glucose branchés ainsi obtenus par :
- au moins une étape de traitement au charbon actif et/ou au noir granulaire,
- au moins une étape de filtration stérilisante, et
- de manière facultative, au moins une étape de traitement thermique,
5) récupération des polymères solubles de glucose branchés purifiés ainsi obtenus.

2. Procédé selon la revendication 1, **caractérisé en ce que** l'étape 4) de purification comprend la succession d'étapes suivantes :
i) amener le pH de la solution aqueuse contenant les polymères solubles de glucose branchés à une valeur comprise entre 4 et 5, de préférence à une valeur de 4,5,
ii) réaliser sur ladite solution ainsi obtenue deux étapes successives de traitement au charbon actif et/ou au noir granulaire,
iii) filtrer sur filtre de diamètre de pore de 10 µm, puis sur filtre de diamètre de pore de 1 µm,
iv) évaporer, concentrer et déminéraliser la solution de polymères solubles de glucose branchés,
v) réaliser une troisième étape de traitement au charbon actif et/ou au noir granulaire,
vi) filtrer sur filtre de diamètre de pore de 10 µm, puis sur filtre de diamètre de pore de 1,2 µm
vii) mettre en œuvre une filtration stérilisante consistant en deux filtrations membranaires de diamètre de pore de 0,45 µm puis 0,22 µm.

3. Polymères solubles de glucose branchés préparés à partir d'amidon présentant une teneur en amylose comprise entre 35 et 80 % en poids, ayant une teneur en liaisons glucosidiques α-1,6 comprise entre 8 et 9 % telle que mesurée par la méthode décrite dans la description, et un Mw compris entre 50.000 et 150.000 daltons tel que mesuré par la méthode décrite dans la description, susceptibles d'être obtenus par le procédé de la revendication 1 ou 2, **caractérisés en ce qu'**ils présentent une résistance à l'alpha amylase pancréatique exprimée selon le test « B » tel que décrit dans la description par :
o une teneur en sucres réducteurs comprise entre 25 et 35 % telle que mesurée par la méthode décrite dans la description, et
o un Mw des produits libérés compris entre 6.000 et 12.000 daltons tel que mesuré par la méthode décrite dans la description, et présentant un profil de distribution des longueurs de chaînes branchées constitué de :
- 25 à 80 % de DP < 10,
- 2 à 10 % de DP > 30
avec un DP moyen compris entre 4 et 12.

4. Polymères selon la revendication 3, **caractérisés en ce qu'**ils présentent une osmolalité, déterminée selon un test A, d'une valeur inférieure à 100 mOsm/kg, le test A consistant à déterminer l'osmolalité d'une solution renfermant 100 g sec desdits polymères de glucose branchés placés dans 1 kg d'eau, à l'aide d'un osmomètre MARK 3 de FISKE^{®} ASSOCIATES.

5. Polymères selon l'une ou l'autre des revendications 3 et 4, **caractérisés en ce qu'**ils présentent une résistance à l'alpha amylase pancréatique exprimée selon le test « B » par :
- une teneur en sucres réducteurs supérieure ou égale à 25 % et inférieure à 30 %,
- un Mw des produits libérés supérieur ou égal à 9.500 et inférieur à 12.000 daltons.

6. Polymères selon la revendication 5, **caractérisés en ce qu'**ils présentent une osmolalité, déterminée selon un test « A », d'une valeur comprise entre 10 et 20 mOsm/kg.

7. Polymères selon l'une ou l'autre des revendications 5 et 6, **caractérisés en ce qu'**ils présentent un profil de distribution des longueurs de chaînes branchées constitué de :
- 25 à 40 % de DP < 10,
- 5 à 10 % de DP > 30,
avec un DP moyen compris entre 10 et 12.

8. Polymères selon l'une ou l'autre des revendications 3 et 4, **caractérisés en ce qu'**ils présentent une résistance à l'alpha amylase pancréatique exprimée selon le test « B » par :
- une teneur en sucres réducteurs supérieure ou égale à 30 % et inférieure à 35 %,
- un Mw des produits libérés supérieur ou égal à 6.000 daltons et inférieur à 9.500 daltons.

9. Polymères selon la revendication 8, **caractérisés en ce qu'**ils présentent une osmolalité, déterminée selon un test « A », d'une valeur comprise entre 50 et 100 mOsm/kg.

10. Polymères selon l'une ou l'autre des revendications 8 et 9, **caractérisés en ce qu'**ils présentent un profil de distribution des longueurs de chaînes branchées constitué de :
- 65 à 80 % de DP < 10,
- 2 à 5 % de DP > 30,
avec un DP moyen compris entre 4 et 6.

11. Compositions destinées à être utilisées chez l'homme et l'animal dans des applications alimentaires et pharmaceutiques, **caractérisées en ce qu'**elles contiennent les polymères de glucose branchés selon l'une quelconque des revendications 3 à 10 ou susceptibles d'être obtenus par le procédé selon les revendications 1 et 2.

12. Solution pour dialyse péritonéale, **caractérisée en ce qu'**elle comprend, en tant qu'agent osmotique, au moins un polymère soluble branché selon l'une quelconque des revendications 3 à 10.

13. Solution pour dialyse péritonéale selon la revendication 12, **caractérisée en ce qu'**elle comprend en outre un polyol choisi dans le groupe formé par le sorbitol, le mannitol, le maltitol, le xylitol, l'érythritol et les hydrolysats d'amidon hydrogénés.

14. Solution pour dialyse péritonéale selon la revendication 12, **caractérisée en ce qu'**elle comprend en outre des agents tampons tels que les sels de lactate, d'acétate et de gluconate.

15. Solution pour dialyse péritonéale selon l'une quelconque des revendications 12 à 14, **caractérisée en ce qu'**elle comprend en outre du glucose, du maltose et/ou des polymères de glucose.

16. Solution pour dialyse péritonéale selon l'une quelconque des revendications 12 à 15, **caractérisée en ce qu'**elle comprend en outre des acides aminés et du glucose.

## Patentansprüche

1. Verfahren zur Herstellung von löslichen verzweigten Glucosepolymeren, **dadurch gekennzeichnet, dass** es die folgende Abfolge von Schritten umfasst:
1) Aufbrechen und Solubilisieren einer Stärkemilch mit einem Amylosegehalt zwischen einschließlich 35 und 80 Gew.-% und einer Trockenmasse zwischen einschließlich 5 und 25 %,
2) kontinuierliche Zugabe eines Glykogen-Verzweigungsenzyms, das aus thermophilen Mikroorganismen extrahiert wurde und eine Aktivität zwischen einschließlich 25 000 U/ml und 60 000 U/ml aufweist, in einer Dosis zwischen einschließlich 800 und 2.500 U/g Trockenstärke, bei einer Temperatur zwischen einschließlich 60 °C und 80 °C, für eine Dauer von 12 bis 18 Stunden,
3) wahlweise Behandlung mit einem Stärkeverflüssigungsenzym vom Typ α-Amylase mit einer Aktivität zwischen einschließlich 100 und 150 KNU/ml bei einer Temperatur von 95°C, einem pH-Wert zwischen einschließlich 5 und 8, für 30 bis 60 Minuten, vorzugsweise für 45 Minuten,
4) Reinigung der so erhaltenen löslichen verzweigten Glucosepolymere durch:
- mindestens einen Behandlungsschritt mit Aktivkohle und/oder körnigem Ruß,
- mindestens einen sterilisierenden Filtrationsschritt und
- wahlweise mindestens einen Wärmebehandlungsschritt,
5) Gewinnung der so erhaltenen gereinigten löslichen verzweigten Glucosepolymere.

2. Verfahren nach Anspruch 1, **dadurch gekennzeichnet, dass** der Reinigungsschritt 4) die folgende Abfolge von Schritten umfasst:
i) Einstellen des pH-Werts der wässrigen Lösung, die die löslichen verzweigten Glucosepolymere enthält, auf einen Wert zwischen einschließlich 4 und 5, vorzugsweise auf einen Wert von 4,5,
ii) Durchführen von zwei aufeinanderfolgenden Behandlungsschritten mit Aktivkohle und/oder körnigem Ruß an der so erhaltenen Lösung,
iii) Filtrieren über einen Filter mit einem Porendurchmesser von 10 µm und anschließend über einen Filter mit einem Porendurchmesser von 1 µm,
iv) Eindampfen, Konzentrieren und Entmineralisieren der Lösung von löslichen verzweigten Glucosepolymeren,
v) Durchführen eines dritten Behandlungsschritts mit Aktivkohle und/oder körnigem Ruß,
vi) Filtrieren über einen Filter mit einem Porendurchmesser von 10 µm und anschließend über einen Filter mit einem Porendurchmesser von 1,2 µm,
vii) Durchführen einer sterilisierenden Filtration, die aus zwei Membranfiltrationen mit einem Porendurchmesser von 0,45 µm und dann 0,22 µm besteht.

3. Lösliche verzweigte Glucosepolymere, hergestellt aus Stärke mit einem Amylosegehalt zwischen einschließlich 35 und 80 Gew.-%, einem Gehalt an α-1,6-glucosidischen Bindungen zwischen einschließlich 8 und 9 %, gemessen nach der in der Beschreibung beschriebenen Methode, und einem Mw zwischen einschließlich 50 000 und 150 000 Dalton, gemessen nach der in der Beschreibung beschriebenen Methode,
die durch das Verfahren nach Anspruch 1 oder 2 erhalten werden können, **dadurch gekennzeichnet, dass** sie eine Resistenz gegen pankreatische alpha-Amylase aufweisen, die gemäß dem Test "B", wie in der Beschreibung beschrieben, ausgedrückt wird durch:
o einen Gehalt an reduzierenden Zuckern zwischen einschließlich 25 und 35 %, gemessen nach der in der Beschreibung beschriebenen Methode, und
o ein Mw der freigesetzten Produkte zwischen einschließlich 6 000 und 12 000 Dalton, gemessen nach der in der Beschreibung beschriebenen Methode, und ein Verteilungsprofil der Längen der verzweigten Ketten aufweisen, das gebildet wird aus:
- 25 bis 80 % DP < 10,
- 2 bis 10 % DP > 30
mit einem mittleren DP zwischen einschließlich 4 und 12.

4. Polymere nach Anspruch 3, **dadurch gekennzeichnet, dass** sie eine Osmolalität, bestimmt nach einem Test A, von weniger als 100 mOsm/kg aufweisen, wobei der Test A darin besteht, die Osmolalität einer Lösung, die 100 g Trockenmasse der verzweigten Glukosepolymere in 1 kg Wasser enthält, mit Hilfe eines Osmometers MARK 3 von FISKE^{®} ASSOCIATES zu bestimmen.

5. Polymere nach einem der Ansprüche 3 und 4, **dadurch gekennzeichnet, dass** sie eine Resistenz gegen pankreatische alpha-Amylase aufweisen, die gemäß dem Test "B" ausgedrückt wird durch:
- einen Gehalt an reduzierenden Zuckern von mindestens 25 % und weniger als 30 %,
- ein Mw der freigesetzten Produkte von mindestens 9 500 und weniger als 12 000 Dalton.

6. Polymere nach Anspruch 5, **dadurch gekennzeichnet, dass** sie eine Osmolalität, bestimmt nach einem Test "A", zwischen einschließlich 10 und 20 mOsm/kg aufweisen.

7. Polymere nach einem der Ansprüche 5 und 6, **dadurch gekennzeichnet, dass** sie ein Verteilungsprofil der Längen der verzweigten Ketten aufweisen, das gebildet wird aus:
- 25 bis 40 % DP < 10,
- 5 bis 10 % DP > 30,
mit einem mittleren DP zwischen einschließlich 10 und 12.

8. Polymere nach einem der Ansprüche 3 und 4, **dadurch gekennzeichnet, dass** sie eine Resistenz gegen pankreatische alpha-Amylase aufweisen, die gemäß dem Test "B" ausgedrückt wird durch:
- einen Gehalt an reduzierenden Zuckern von mindestens 30 % und weniger als 35 %,
- ein Mw der freigesetzten Produkte von mindestens 6 000 Dalton und weniger als 9 500 Dalton.

9. Polymere nach Anspruch 8, **dadurch gekennzeichnet, dass** sie eine Osmolalität, bestimmt nach einem Test "A", zwischen einschließlich 50 und 100 mOsm/kg aufweisen.

10. Polymere nach einem der Ansprüche 8 und 9, **dadurch gekennzeichnet, dass** sie ein Verteilungsprofil der Längen der verzweigten Ketten aufweisen, das gebildet wird aus:
- 65 bis 80 % DP < 10,
- 2 bis 5 % DP > 30,
mit einem mittleren DP zwischen einschließlich 4 und 6.

11. Zusammensetzungen zur Verwendung bei Menschen und Tieren in Lebensmitteln und pharmazeutischen Anwendungen, **dadurch gekennzeichnet, dass** sie verzweigte Glucosepolymere gemäß einem der Ansprüche 3 bis 10 enthalten oder Glucosepolymere, die durch das Verfahren gemäß den Ansprüchen 1 und 2 erhältlich sind.

12. Lösung für die Peritonealdialyse, **dadurch gekennzeichnet, dass** sie als osmotisches Mittel mindestens ein lösliches verzweigtes Polymer nach einem der Ansprüche 3 bis 10 enthält.

13. Peritonealdialyselösung nach Anspruch 12, **dadurch gekennzeichnet, dass** sie zusätzlich ein Polyol aus der Gruppe Sorbitol, Mannitol, Maltitol, Xylitol, Erythritol und hydrierte Stärkehydrolysate enthält.

14. Peritonealdialyselösung nach Anspruch 12, **dadurch gekennzeichnet, dass** sie zusätzlich Puffermittel wie Lactat-, Acetat- und Gluconatsalze enthält.

15. Peritonealdialyselösung nach einem der Ansprüche 12 bis 14, **dadurch gekennzeichnet, dass** sie zusätzlich Glucose, Maltose und/oder Glucosepolymere enthält.

16. Peritonealdialyselösung nach einem der Ansprüche 12 bis 15, **dadurch gekennzeichnet, dass** sie zusätzlich Aminosäuren und Glucose enthält.

## Claims

1. A method for preparing soluble branched glucose polymers, **characterised in that** it comprises the succession of following steps:
1) bursting and solubilisation of a starch milk having an amylose content comprised between 35 and 80% by weight, and a dry matter comprised between 5 and 25%,
2) continuous addition of a glycogen branching enzyme extracted from thermophilic microorganisms, having an activity comprised between 25,000 U/ml and 60,000 U/ml, at a dose comprised between 800 and 2,500 U/g dry starch, at a temperature comprised between 60°C and 80°C, for a period of 12 to 18 hours,
3) optionally, treatment with an α-amylase type starch liquefaction enzyme having an activity comprised between 100 and 150 KNU/ml at a temperature of 95°C, a pH comprised between 5 and 8, for 30 to 60 minutes, preferably for 45 minutes,
4) purification of the soluble branched glucose polymers thus obtained by:
- at least one step of treatment with activated carbon and/or with granular black carbon,
- at least one sterilising filtration step, and
- optionally at least one heat treatment step,
5) recovery of the soluble purified branched glucose polymers thus obtained.

2. The method according to claim 1, **characterised in that** step 4) of purification comprises the succession of following steps:
i) bringing the pH of the aqueous solution containing the soluble branched glucose polymers to a value comprised between 4 and 5, preferably to a value of 4.5,
ii) carrying out, on said solution thus obtained, two successive steps of treatment with activated carbon and/or with granular black carbon,
iii) filtering through a 10 µm pore diameter filter, then through a 1 µm pore diameter filter,
iv) evaporating, concentrating and demineralising the solution of soluble branched glucose polymers,
v) carrying out a third step of treatment with activated carbon and/or with granular black carbon,
vi) filtering through a 10 µm pore diameter filter, then through a 1.2 µm pore diameter filter,
vii) implementing a sterilising filtration consisting of two membrane filtrations with a pore diameter of 0.45 µm then 0.22 µm.

3. Soluble branched glucose polymers prepared from starch having an amylose content comprised between 35 and 80% by weight, having a content of α-1,6 glucoside linkages comprised between 8 and 9% as measured by the method described in the description,
and a Mw comprised between 50,000 and 150,000 daltons as measured by the method described in the description,
likely to be obtained by the method of claim 1 or 2, **characterised in that** they have a resistance to pancreatic alpha amylase expressed according to the test "B" as described in the description by:
∘ a reducing sugar content comprised between 25 and 35% as measured by the method described in the description, and
∘ a Mw of the released products comprised between 6,000 and 12,000 daltons as measured by the method described in the description,
and having a branched chain length distribution profile consisting of:
- 25 to 80% of DP < 10,
- 2 to 10% of DP > 30
with an average DP comprised between 4 and 12.

4. The polymers according to claim 3, **characterised in that** they have an osmolality, determined according to a test A, of a value which is less than 100 mOsm/kg, the test A consisting in determining the osmolality of a solution containing 100 g dry of said branched glucose polymers placed in 1 kg of water, using a FISKE^{®} ASSOCIATES MARK 3 osmometer.

5. The polymers according to either of claims 3 or 4, **characterised in that** they have a resistance to pancreatic alpha amylase expressed according to the test "B" by:
- a reducing sugar content which is greater than or equal to 25% and less than 30%,
- a Mw of the released products which is greater than or equal to 9,500 and less than 12,000 daltons.

6. The polymers according to claim 5, **characterised in that** they have an osmolality, determined according to a test "A", of a value comprised between 10 and 20 mOsm/kg.

7. The polymers according to either of claims 5 or 6, **characterised in that** they have a branched chain length distribution profile consisting of:
- 25 to 40% of DP < 10,
- 5 to 10% of DP > 30,
with an average DP comprised between 10 and 12.

8. The polymers according to either of claims 3 or 4, **characterised in that** they have a resistance to pancreatic alpha amylase expressed according to the test "B" by:
- a reducing sugar content which is greater than or equal to 30% and less than 35%,
- a Mw of the released products which is greater than or equal to 6,000 daltons and less than 9,500 daltons.

9. The polymers according to claim 8, **characterised in that** they have an osmolality, determined according to a test "A", of a value comprised between 50 and 100 mOsm/kg.

10. The polymers according to either of claims 8 or 9, **characterised in that** they have a branched chain length distribution profile consisting of:
- 65 to 80% of DP < 10,
- 2 to 5% of DP > 30,
with an average DP comprised between 4 and 6.

11. Compositions intended to be used in humans and animals in food and pharmaceutical applications, **characterised in that** they contain the branched glucose polymers according to any one of claims 3 to 10 or likely to be obtained by the method according to claims 1 and 2.

12. A solution for peritoneal dialysis, **characterised in that** it comprises, as osmotic agent, at least one soluble branched polymer according to any one of claims 3 to 10.

13. The solution for peritoneal dialysis according to claim 12, **characterised in that** it further comprises a polyol selected from the group formed by sorbitol, mannitol, maltitol, xylitol, erythritol and hydrogenated starch hydrolysates.

14. The solution for peritoneal dialysis according to claim 12, **characterised in that** it further comprises buffering agents such as lactate, acetate and gluconate salts.

15. The solution for peritoneal dialysis according to any one of claims 12 to 14, **characterised in that** it further comprises glucose, maltose and/or glucose polymers.

16. The solution for peritoneal dialysis according to any one of claims 12 to 15, **characterised in that** it further comprises amino acids and glucose.
